(19) Europäisches Patentamt
European Patent Office
Office européen des brevets

(11) Veröffentlichungsnummer: **0 468 258 B1**

(12) **EUROPÄISCHE PATENTSCHRIFT**

(45) Veröffentlichungstag der Patentschrift: **02.11.95**

(51) Int. Cl.$^6$: **C07D 498/04**, A61K 31/535, A61K 31/66, C07F 9/6561

(21) Anmeldenummer: **91111273.8**

(22) Anmeldetag: **06.07.91**

Die Akte enthält technische Angaben, die nach dem Eingang der Anmeldung eingereicht wurden und die nicht in dieser Patentschrift enthalten sind.

# (54) Substituierte Pyrido-oxazine.

(30) Priorität: **21.07.90 DE 4023308**

(43) Veröffentlichungstag der Anmeldung:
**29.01.92 Patentblatt 92/05**

(45) Bekanntmachung des Hinweises auf die Patenterteilung:
**02.11.95 Patentblatt 95/44**

(84) Benannte Vertragsstaaten:
**AT BE CH DE DK ES FR GB GR IT LI LU NL SE**

(56) Entgegenhaltungen:
**DE-A- 3 817 298**
**US-A- 3 838 120**

**CHEMICAL ABSTRACTS, Band 111, Nr. 15, 9. Oktober 1989, Columbus, Ohio, USA P.CANONNE et al. "Spirocyclization of 1-(o-aminophenyl)cycloalkanols and 1-(2-amino-3-pyridinyl)cycloalkanols" Seite 753, linke Spalte, Zusammenfassung-Nr. 134 061q**

(73) Patentinhaber: **BAYER AG**

**D-51368 Leverkusen (DE)**

(72) Erfinder: **Hübsch, Walter, Dr.**
**Am Eckbusch 43/50**
**W-5600 Wuppertal 12 (DE)**
Erfinder: **Angerbauer, Rolf, Dr.**
**Moospfad 20**
**W-5600 Wuppertal 1 (DE)**
Erfinder: **Fey, Peter, Dr.**
**Ursulastrasse 14**
**W-5600 Wuppertal 1 (DE)**
Erfinder: **Bischoff, Hilmar, Dr.**
**Am Rohm 78**
**W-5600 Wuppertal 1 (DE)**
Erfinder: **Bender, Joachim, Dr.**
**4th Parker Street**
**Berkeley CA 94710 (US)**
Erfinder: **Schmidt, Delf, Dr.**
**Am Eckbusch 55b**
**W-5600 Wuppertal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft substituierte Pyrido-oxazine, Zwischenverbindungen zu ihrer Herstellung, ihre Herstellung und ihre Verwendung in Arzneimitteln.

Es ist bekannt, daß aus Pilzkulturen isolierte Lactonderivate. Inhibitoren der 3-Hydroxy-3-methyl-glutaryl Coenzym A Reduktase (HMG-CoA-Reduktase) sind [Mevinolin, EP 22 478; US 4 231 938].

Ebenso sind aus dem Stand der Technik HMG-CoA-Reduktase inhibitorische Endihydroxycarbonsäurederivate bzw. deren ringgeschlossene lactonische Analoga bekannt, die unterschiedliche heterozyclische Grundsysteme enthalten. So offenbart beispielsweise EP-A-0 247 633 entsprechend substituierte über das Ring-Stickstoffatom gebundene Pyrrole, EP-A-0 2 372 500 entsprechend substituierte Pyrazolo pyridine und EP-A-0 3 06 929 entsprechend substituierte Pyridine.

Außerdem werden in der DE 38 17 298 A1 phosphorhaltige HMG-CoA-Reduktase-Inhibitoren mit antihypercholesterolemischer Aktivität publiziert.

Es wurden neue substituierte Pyrido-oxazine der allgemeinen Formel (I)

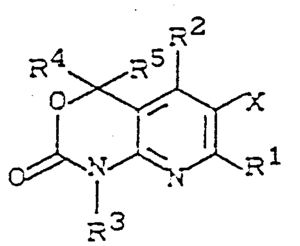

(I)

in welcher

$R^1$
- für geradkettiges oder verzweigtes Alkyl mit bis zu 6 Kohlenstoffatomen steht,
- für Cyclopropyl, Cyclopentyl oder Cyclohexyl steht,

$R^2$
- für Phenyl steht, das gegebenenfall durch geradkettiges oder verzweigtes Alkyl mit bis zu 4 Kohlenstoffatomen, Trifluormethyl, Fluor, Chlor oder Brom substituiert ist,

$R^3$
- für Wasserstoff steht oder
- für geradkettiges oder verzweigtes Alkyl mit bis zu 8 Kohlenstoffatomen steht, das gegebenenfalls durch Cyano, Alkoxy mit bis zu 4 Kohlenstoffatomen, Phenyl oder Pyridyl substituiert ist,
- für geradkettiges oder verzweigtes Alkenyl mit bis zu 6 Kohlenstoffatomen seth,
- für Cyclopropyl, Cyclopentyl oder Cyclohexyl steht,

$R^4$ und $R^5$ für Wasserstoff stehen,

X
- für einen Rest der Formel -A-B steht, worin

A
- eine Gruppe der Formel $-CH_2-CH_2-$ oder $-CH=CH-$bedeutet,

B
- eine Gruppe der Formel

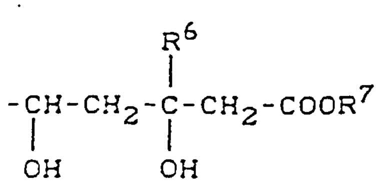

oder

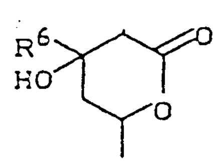

bedeutet, worin

$R^6$

- Wasserstoff bedeutet
  und

$R^7$

- Wasserstoff oder geradkettiges oder verzweigtes Alkyl mit bis zu 8 Kohlenstoffatomen oder Benzyl bedeutet, oder
- Phenyl oder ein Kation bedeutet

und deren Salze gefunden.

Bildet $R^7$ mit der Carboxygruppe einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu gehören beispielsweise Alkylester ($C_1$ bis $C_6$) und Aralkylester ($C_7$ bis $C_{10}$), bevorzugt ($C_1$-$C_4$)-Alkylester sowie Benzylester. Darüberhinaus seien die folgenden Esterreste genannt: Methylester, Ethylester, Propylester, Benzylester.

Steht $R^7$ für ein Kation, so ist bevorzugt ein physiologisch verträgliches Metall- oder Ammoniumkation gemeint. Bevorzugt sind hierbei Alkali- bzw. Erdalkalikationen wie beispielsweise Lithium-, Natrium-, Kalium-, Magnesium- oder Calciumkationen, sowie Aluminium- oder Ammoniumkationen, sowie nichttoxische substituierte Ammoniumkationen aus Aminen wie ($C_1$-$C_4$)-Dialkylamine, ($C_1$-$C_4$)-Trialkylamine, Prokain, Dibenzylamin, N,N'-Dibenzylethylendiamin, N-Benzyl-$\beta$-phenylethylamin, N-Methylmorpholin oder N-Ethylmorpholin, 1-Ephenamin, Dihydroabietylamin, N,N'-Bis-dihydroabietylethylendiamin, N-Niederalkylpiperidin und andere Amine, die zur Bildung von Salzen verwendet werden können.

Ebenso kann $R^8$ für eines der oben aufgeführten physiologisch verträglichen Metall- oder Ammoniumkationen stehen.

Überraschenderweise zeigen die erfindungsgemäßen substituierten Pyrido-oxazine eine überlegene inhibitorische Wirkung auf die HMG-CoA-Reduktase (3-Hydroxy-3-methyl-glutaryl-Coenzym A Reduktase).

Bevorzugt sind Verbindungen der allgemeinen Formel (I), in welcher

$R^1$

- für geradkettiges oder verzweigtes Alkyl mit bis zu 4 Kohlenstoffatomen oder Cyclopropyl steht,

$R^2$

- für Phenyl steht, das gegebenenfalls durch Methyl, Trifluormethyl, Fluor oder Chlor substituiert ist,

$R^3$

- für Wasserstoff steht oder
- für geradkettiges oder verzweigtes Alkyl mit bis zu 6 Kohlenstoffatomen steht, das gegebenenfalls durch Cyano, Phenyl oder Pyridyl substituiert ist,
- für geradkettiges oder verzweigtes Alkenlyl mit bis zu 4 Kohlenstoffatomen steht,

$R^4$ und $R^5$ für Wasserstoff stehen,

X

- für einen Rest der Formel -A-B steht,
  worin

A

- die -CH=CH- oder $CH_2$-$CH_2$-Gruppe bedeutet,

B

- eine Gruppe der Formel

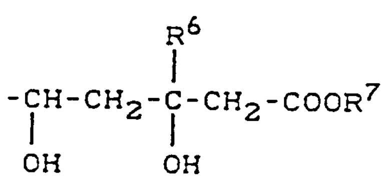

oder

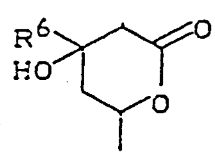

bedeutet,
worin

$R^6$

- Wasserstoff bedeutet
  und

$R^7$

- Wasserstoff, Methyl, Ethyl, Propyl, Isopropyl, Butyl, Isobutyl, tert.Butyl oder Benzyl bedeutet, oder
- ein Natrium-, Kalium-, Calcium-, Magnesium- oder Ammoniumion bedeutet

und deren Salze.

Die erfindungsgemäßen substituierten Pyrido-oxazine der allgemeinen Formel (I) haben mehrere asymmetrische Kohlenstoffatome und können daher in verschiedenen stereochemischen Formen existieren, Sowohl die einzelnen Isomeren als auch deren Mischungen sind Gegenstand der Erfindung.

Es wurde ein Verfahren zur Herstellung der substituierten Pyrido-oxazine der allgemeinen Formel (I)

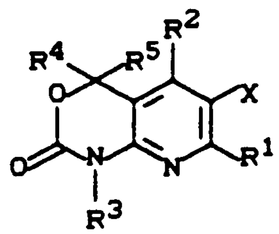

(I)

in welcher
$R^1$, $R^2$, $R^3$, $R^4$ und $R^5$ die oben angegebene Bedeutung haben,
gefunden, das dadurch gekennzeichnet ist, daß man Ketone der allgemeinen Formel (VIII)

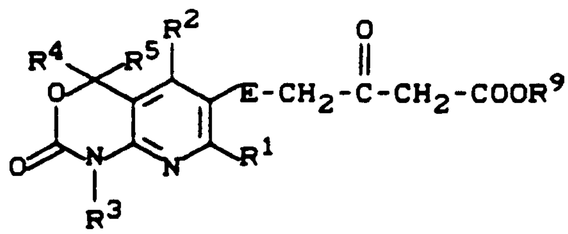

(VIII)

in welcher
$R^1$, $R^2$, $R^3$, $R^4$ und $R^5$ die oben angegebene Bedeutung haben,

$R^9$

- für Alkyl steht,

und

E für eine Gruppe

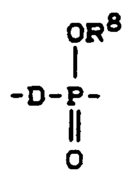 (VIIIa) oder 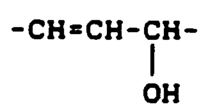 (VIIIb)

reduziert,
im Fall der Herstellung der Säuren die Ester verseift,
im Fall der Herstellung der Lactone die Carbonsäuren cyclisiert,
im Fall der Herstellung der Ethylenverbindungen (A = $-CH_2-CH_2-$) die Ethenverbindungen (A = -CH=CH-) nach üblichen Methoden hydriert,
und gegebenenfalls Isomeren trennt.

Das erfindungsgemäße Verfahren kann durch das folgende Formelschema erläutert werden:

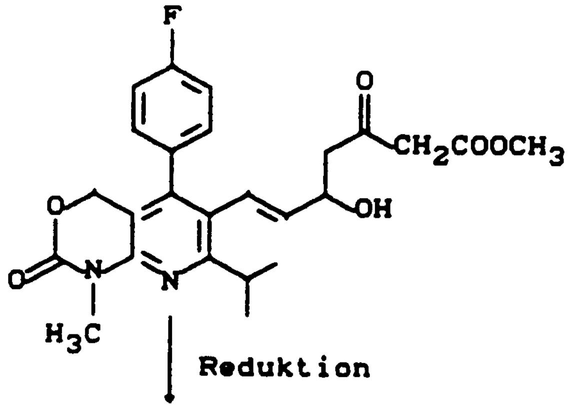

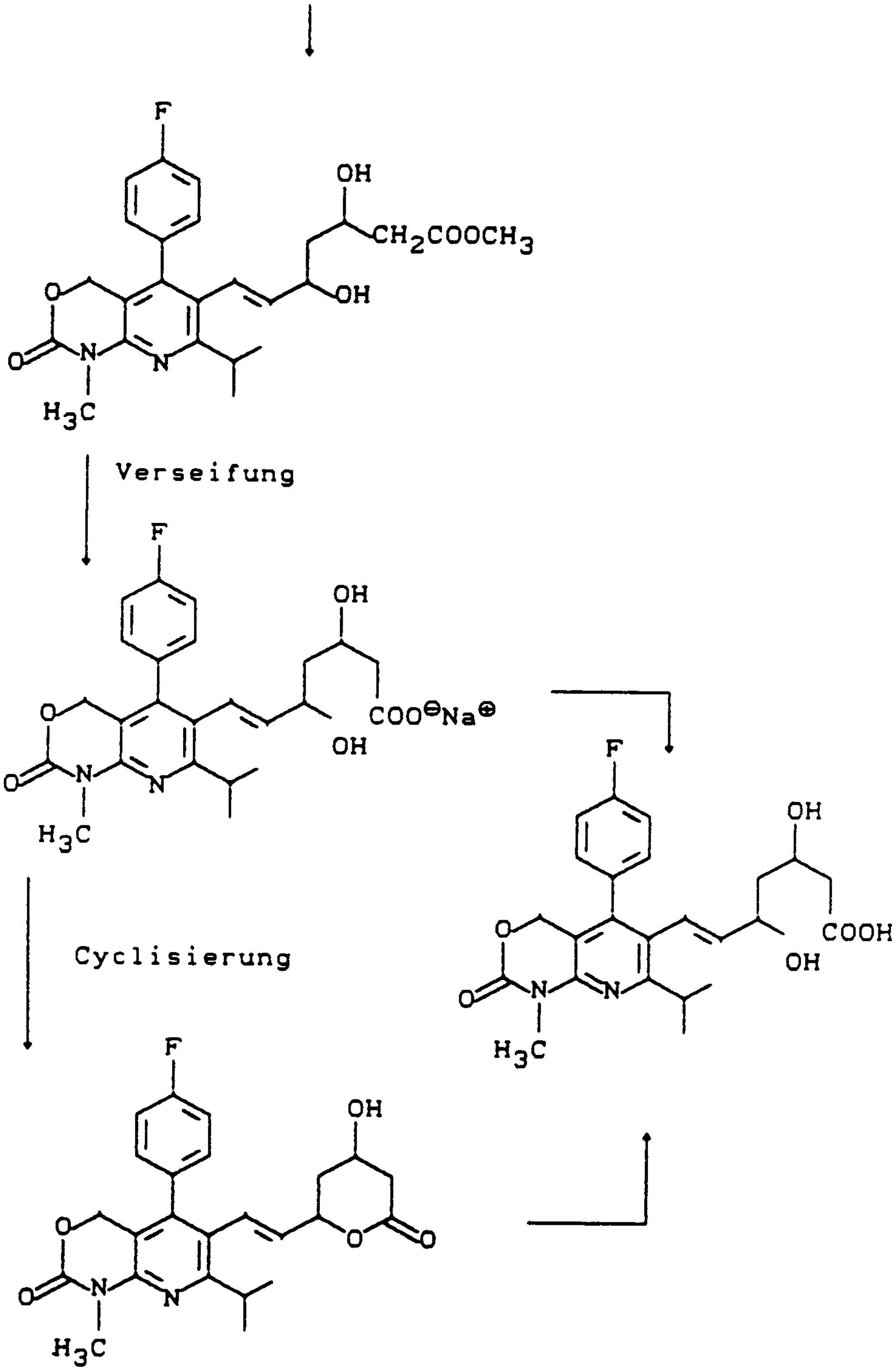


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranaten, Natrium-trialkyl-hydrido-boranaten, Natrium-cyano-trihydrido-boranat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan,

Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Im Fall, daß E für den Rest der Formel (VIIIa) steht, werden Alkohole, wie Methanol, Ethanol oder Propanol, vorzugsweise Ethanol, eingesetzt.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden.

Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80 °C bis +30 °C, bevorzugt von -78 °C bis 0 °C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen A für eine Ethylengruppierung steht, kann die Reduktion der Ketone (VIII) unter solchen Bedingungen durchgeführt werden, unter denen sowohl die Carbonylgruppe als auch die Doppelbindung reduziert wird.

Darüber hinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ia)

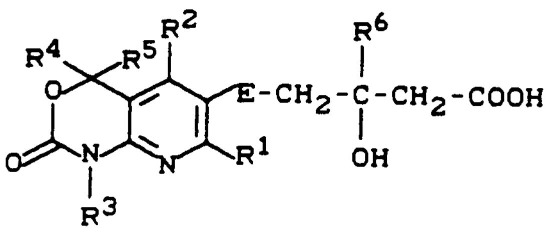

(Ia)

in welcher
$R^1$, $R^2$, $R^3$, $R^4$, $R^5$, $R^6$ und E die oben angegebene Bedeutung haben.

Die Carbonsäureester im Rahmen der allgemeinen Formel (I) entsprechen der Formel (Ib)

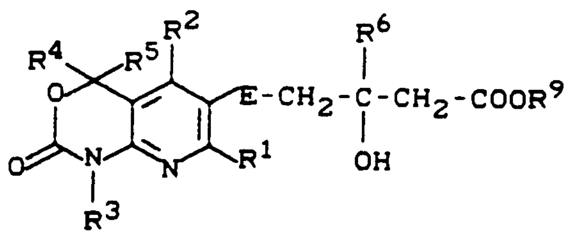

(Ib)

in welcher $R^1$, $R^2$, $R^3$, $R^4$, $R^5$, $R^6$ und E die oben angegebene Bedeutung haben,
und

$R^9$ - für Alkyl steht.

Die Salze der erfindungsgemäßen Verbindungen im Rahmen der allgemeinen Formel (I) entsprechen der Formel (Ic)

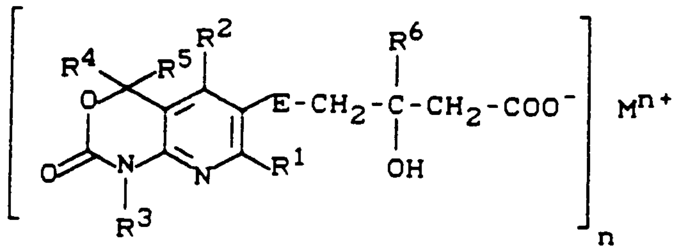

(Ic)

in welcher
$R^1$, $R^2$, $R^3$, $R^4$, $R^5$, $R^6$ und E die oben angegebene Bedeutung haben,
und
$M^{n+}$ für ein Kation steht.

Die Lactone im Rahmen der allgemeinen Formel (I) entsprechen der Formel (Id)

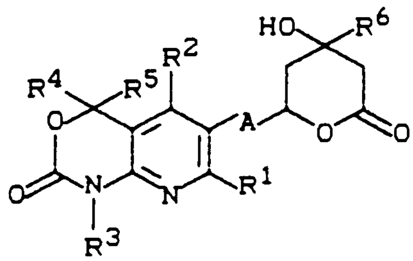

(Id)

in welcher
$R^1$, $R^2$, $R^3$, $R^4$, $R^5$, $R^6$ und A die oben angegebene Bedeutung haben.

Zur Herstellung der erfindungsgemäßen Carbonsäuren der allgemeinen Formel (Ia) werden im allgemeinen die Carbonsäureester der allgemeinen Formel (Ib) oder die Lactone der allgemeinen Formel (Id) nach üblichen Methoden verseift Die Verseifung erfolgt im allgemeinen indem man die Ester oder die Lactone in inerten Lösemitteln mit üblichen Basen behandelt, wobei im allgemeinen zunächst die Salze der allgemeinen Formel (Ic) entstehen, die anschließend in einem zweiten Schritt durch Behandeln mit Säure in die freien Säuren der allgemeinen Formel (Ia) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ic) als Zwischenprodukte, die isoliert werden können. Die erfindungsgemäßen Säuren (Ia) erhält man durch Behandeln der Salze (Ic) mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ia) hierbei als vorteilhaft erwiesen, die

basische Reaktionsmischung der Verseifung in einem zweiten Schritt ohne Isolierung der Salze anzusäuren. Die Säuren können dann in üblicher Weise isoliert werden

Zur Herstellung der erfindungsgemäßen Lactone der Formel (Id) werden im allgemeinen die erfindungsgemäßen Carbonsäuren (Ib) nach üblichen Methoden cyclisiert. beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40 °C bis +200 °C, bevorzugt von -25 °C bis +50 °C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N'-Dicyclohexylcarbodiimid, N-Cyclohexyl-N'-[2-(N''-methylmorpholinium)ethyl]carbodiimid, Paratoluolsulfonat oder N-(3-Dimethylaminopropyl)-N'-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen, Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0 °C bis +80 °C, bevorzugt von +10 °C bis +50 °C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Lactone. Besonders bevorzugt wird hierbei die racemische Mischung der trans-Lactone (VII) durch Behandeln entweder mit D-(+)- oder L-(-)-α-Methylbenzylamin nach üblichen Methoden in die diastereomeren Dihydroxyamide (Ie)

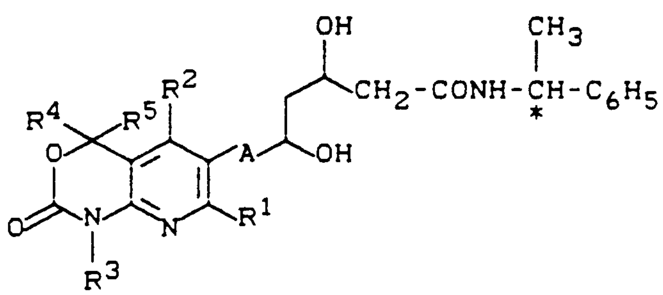

(Ie)

überführt, die anschließend wie üblich durch Chromatographie oder Kristallisation in die einzelnen Diastereomeren getrennt werden können, Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergibt die entsprechenden enantiomerenreinen Dihydroxysäuren (Ia), die wie oben beschrieben durch Cyclisierung in die enantiomerenreinen Lactone überführt werden können, Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

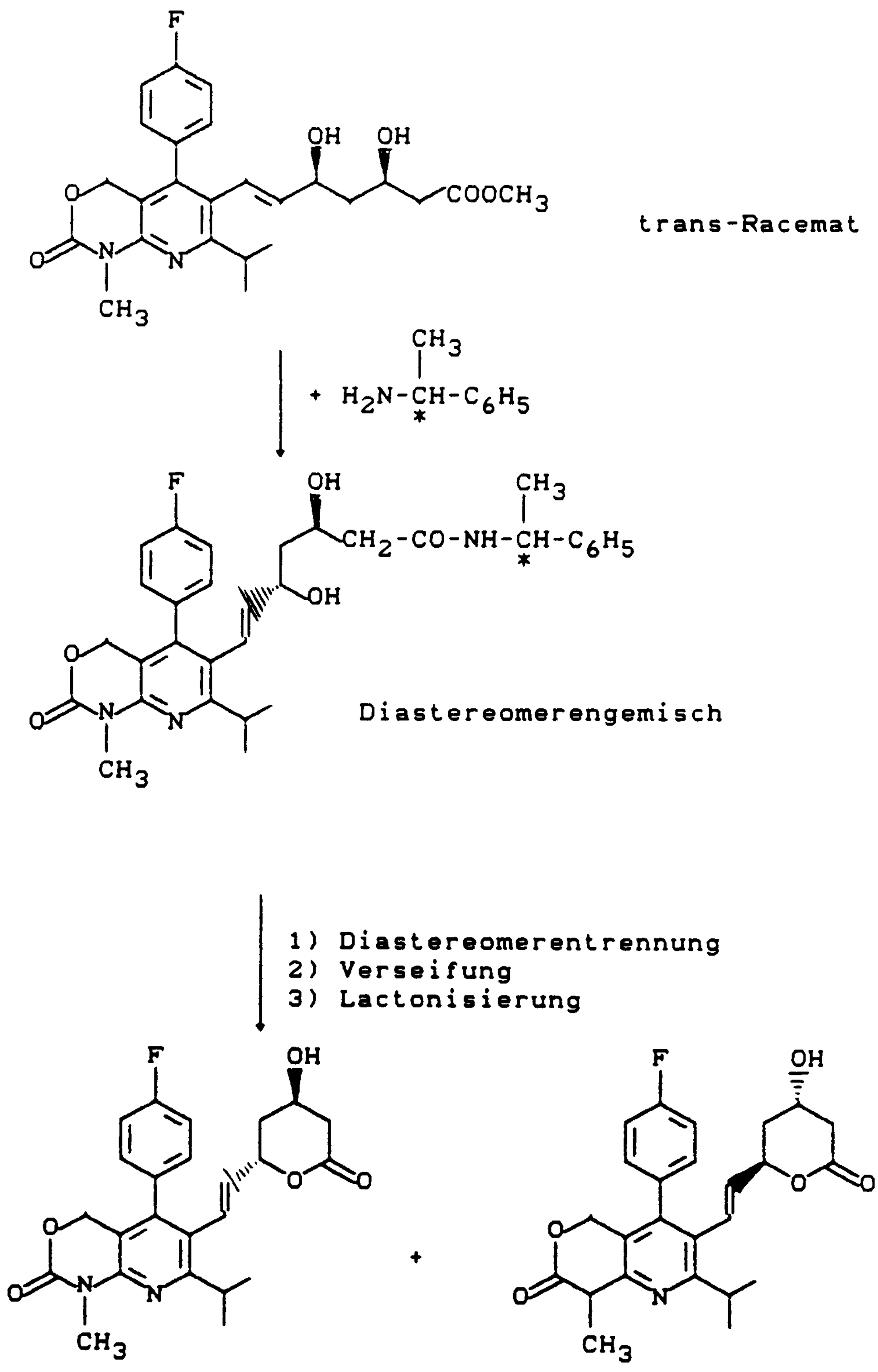


Die als Ausgangsstoffe eingesetzten Ketone (VIII) sind neu.

Die Herstellung der Ketone der Formel (VIIIa) erfolgt in Analogie zu dem in der DE 38 17 298 A1 beschriebenen Verfahren.

Die Herstellung der erfindungsgemäßen Ketone der allgemeinen Formel (VIIIb)

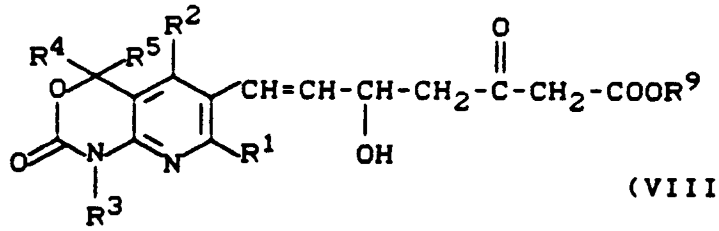

(VIIIb)

in welcher
$R^1$, $R^2$, $R^3$, $R^4$, $R^5$ und $R^9$ die oben angegebene Bedeutung haben,
erfolgt, indem man Aldehyde der allgemeinen Formel (IX)

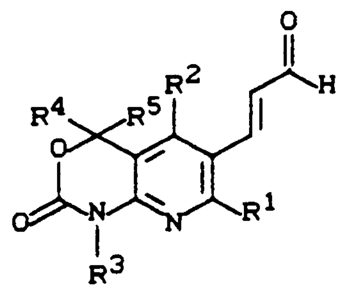

(IX)

in welcher
$R^1$, $R^2$, $R^3$, $R^4$ und $R^5$ die oben angegebene Bedeutung haben,
in inerten Lösemitteln mit Acetessigester der allgemeinen Formel (X)

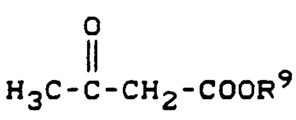

(X)

in welcher
$R^9$ die oben angegebene Bedeutung hat,
in Anwesenheit von Basen umsetzt.

Das erfindungsgemäße Verfahren kann beispielsweise durch folgendes Formelschema erläutert werden:

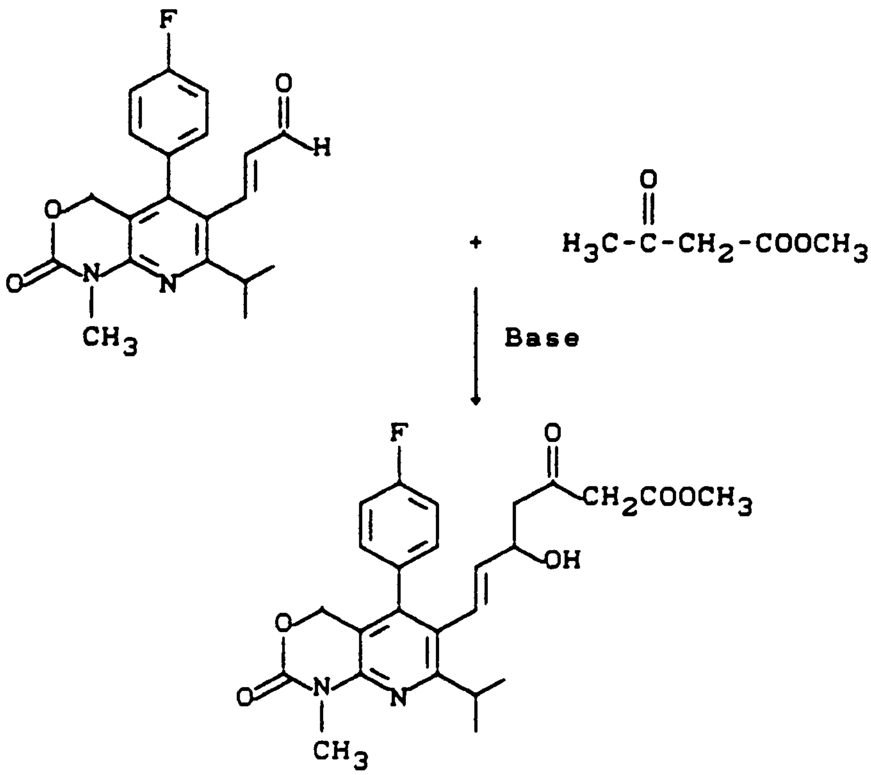


Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80 °C bis +50 °C, bevorzugt von -20 °C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt: Acetessigsäuremethylester, Acetessigsäureethylester, Acetessigsäurepropylester, Acetessigsäureisopropylester.

Die Herstellung der als Ausgangsstoffe eingesetzten Aldehyde der allgemeinen Formel (IX) soll im folgenden beispielhaft für den Fall, daß X für die Gruppe -A-B steht, erläutert werden:

[A]

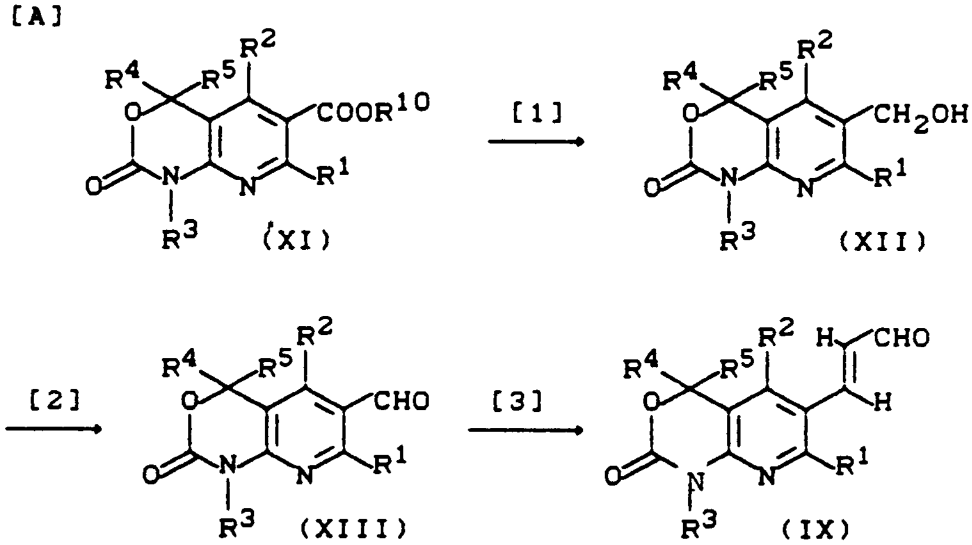


(XI) (XII) (XIII) (IX)

Hierbei werden gemäß Schema A 2H-Pyrido[2,3-d][1,3]oxazine der Formel (XI), in welcher $R^{10}$ für Alkyl mit bis zu 4 Kohlenstoffatomen steht, im ersten Schritt [1] in inerten Lösemitteln wie Toluol oder Ethern, beispielsweise Diethylether, Tetrahydrofuran oder Dioxan, vorzugsweise Tetrahydrofuran oder Toluol, mit Metallhydriden als Reduktionsmittel, beispielsweise Lithiumaluminiumhydrid, Natriumcyanoborhydrid, Natriumaluminiumhydrid, Diisobutylaluminiumhydrid oder Natrium-bis-(2-methoxyethoxy)-dihydroaluminat,in Temperaturbereichen von -70 °C bis +100 °C, vorzugsweise von -70 °C bis Raumtemperatur, bzw. von Raumtemperatur bis 70 °C je nach verwendetem Reduktionsmittel zu den Hydroxymethylverbindungen (XII) reduziert. Vorzugsweise wird die Reduktion mit Diisobutylaluminiumhydrid in Toluol in einem Temperaturbereich von -78 °C bis Raumtemperatur durchgeführt. Die Hydroxymethylverbindungen (XII) werden im zweiten Schritt [2] nach üblichen Methoden zu den Aldehyden (XIII) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0 °C bis 60 °C, bevorzugt bei Raumtemperatur durchgeführt werden, oder aber mit Trifluoressigsäureanhydrid/Dimethylsulfoxid nach den üblichen Methoden der Swern Oxidation durchgeführt werden. Die Aldehyde (XIII) werden im dritten Schritt [3] mit Diethyl-2-(cyclohexylamino)-vinylphosphonat in Anwesenheit von Natriumhydrid in inerten Lösemitteln wie Ethern, beispielsweise Diethylether, Tetrahydrofuran oder Dioxan, vorzugsweise in Tetrahydrofuran, in einem Temperaturbereich von -20 °C bis +40 °C, vorzugsweise von Raumtemperatur bis 70 °C zu den Aldehyden (IX) umgesetzt.

Die hierbei als Ausgangsstoffe eingesetzten 2H-Pyrido[2,3-d][1,3]oxazine (XI) erhält man im Fall, daß $R^4$ und $R^5$ Wasserstoff bedeuten (XIa), gemäß Schema [B], indem man zunächst die Dihydropyridine der Formel (XIV), in welcher $R^{10}$ und $R^{10'}$ gleich oder verschieden sind und die oben angegebene Bedeutung haben, zu den entsprechenden Pyridinen der Formel (XV) oxidiert, anschließend die Esterfunktion ($COOR^{10'}$) durch Reduktion in die entsprechenden Hydroxymethylverbindungen (XVI) überführt und in einem letzten Schritt nach herkömmlichen Methoden cyclisiert und im Fall, daß $R^4$ und/oder $R^5$ verschieden sind von Wasserstoff, beispielsweise die Verbindungen der allgemeinen Formel (XV) zunächst mit Grignard-Verbindungen nach üblicher Methode umsetzt und anschließend cyclisiert.

[B]

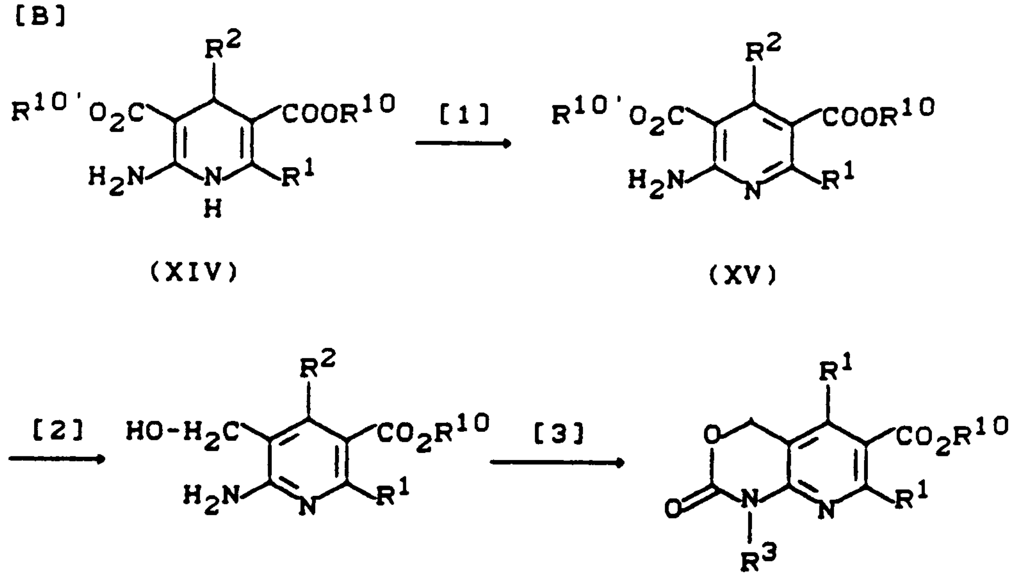


(XIV) (XV)

(XVI) (XIa)

Die Oxidation der Dihydropyridine (XIV) zu den Pyridinen (XV) kann beispielsweise mit Chromoxid in Eisessig in einem Temperaturbereich von -20 °C bis +150 °C, vorzugsweise bei Rückflußtemperatur, oder mit 2,3-Dichlor-5,6-dicyan-p-benzochinon als Oxidationsmittel in inerten Lösemitteln wie Chlorkohlenwasserstoffe, vorzugsweise Methylenchlorid in einem Temperaturbereich von 0 °C bis +100 °C, vorzugsweise bei Raumtemperatur durchgeführt werden.

Die Reduktion zu den Hydroxymethylverbindungen der Formel (XVI) [Schritt 2] wird mit geeigneten Reduktionsmitteln, wie beispielsweise Lithiumaluminiumhydrid, Diisobutylaluminiumhydrid oder Natrium-bis-(2-methoxyethoxy)-dihydroaluminat in inerten Lösemitteln, wie beispielsweise Tetrahydrofuran, durchgeführt.

Die Cyclisierung erfolgt mit Kohlensäurederivaten wie beispielsweise Phosgen, Di- oder Triphosgen, Chlorameisensäureestern oder Kohlensäureestern wie z.B. Diphenylcarbonat unter Zusatz von Basen, wie beispielsweise Alkalimetallhydroxiden, Alkalimetallalkoholaten, tertiären Aminen oder mit 1,8-Diazabicyclo-(5.4.0)-undec-7-en, 1,4-Diazabicyclo[2.2.2]octan oder 1,5-Diazabicyclo[4.3.0]non-5-en. Bevorzugt ist 1,8-Diazabicyclo-(5.4.0)-undec-7-en.

Die Base wird in einer Menge von 1 bis 3 Moläquivalenten, bevorzugt von 1 bis 2 Moläquivalenten eingesetzt.

Die Dihydropyridine der allgemeinen Formel (XIV) sind teilweise neu oder bekannt und können nach bekannten Methoden, beispielsweise durch eine Kondensation von Ethoxycarbonyl-acetamidin-hydrochlorid und (E)-Z-1-(4-Fluorphenyl)-2-methoxycarbonyl-4-methyl-pent-1-en-3-on, hergestellt werden.

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methylglutaryl-Coenzym A (HMG-CoA) Reduktase und infolge dessen Inhibitoren der Cholesterolbiosynthese. Sie können deshalb zur Behandlung von Hyperlipoproteinämie, Lipoproteinämie oder Atherosklerose eingesetzt werden. Die erfindungsgemäßen Wirkstoffe bewirken außerdem eine Senkung des Cholesteringehaltes im Blut.

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Colestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0,03 m Ethylendiamintetraessigsäure, 0,002 m Dithiothreit (SPE)-Puffer pH 7,2, homogenisiert. Anschließend wurde 15 Minuten bei 15 000 g zentrifugiert und das Sediment verworfen, Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes SPE-Puffer aufgenommen, homogenisiert und bei -78 °C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37 °C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase 35 µMol $K_xH_y$-Phosphat PH 7,2, 20 µl Enzympräparation und 56 nMol 3-Hydroxy-3-methyl-glutaryl Coenzym A (Glutaryl-3-$^{14}$C) 100 000 dpm.

Nach Inkubation von 60 Minuten bei 37 °C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. $IC_{50}$-Werte wurden durch Auftrag der prozentualen Hemmung gegen die Konzentration der Verbindung im Test durch Intrapolation bestimmt, Zur Bestimmung der relativen inhibitorischen Potenz wurde der $IC_{50}$-Wert der Referenzsubstanz Mevinolin als 1 gesetzt und mit dem simultan bestimmten $IC_{50}$-Wert der Testverbindung verglichen.

| Bsp.-Nr. | in vitro/rel. $IC_{50}$, Mevinolin = 1 |
|---|---|
| 1a | 3 |
| 1b | 20 |
| 1c | 13 |
| 1d | 66 |
| 1e | 66 |
| 2b | 24 |
| 2e | 50 |

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,0005 bis etwa 20 mg/kg, bevorzugt in Gesamtmengen von etwa 0,001 mg/kg bis 5 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament der Art und Schwere der Erkrankung, der Art der Zubereitung und Applikation, sowie dem Zeitpunkt bzw. Intervall, zu welchem die Verabreichung erfolgt.

Ausgangsverbindungen

Beispiel I

(E)-Z-1-(4-Fluorphenyl)-2-methoxycarbonyl-4-methyl-pent-1-en-3-on

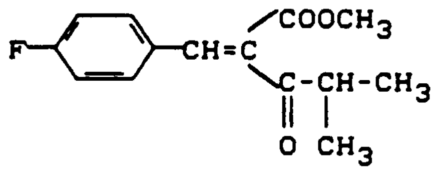

Zu 576,7 g (4 mol) Isobutyrylessigsäuremethylester und 496,5 g (4 mol) 4-Fluorbenzaldehyd in 1 l Isopropanol gibt man eine Lösung von 22,5 ml (0,227 mol) Piperidin und 13,5 ml (0,236 mol) Essigsäure in 100 ml Isopropanol. Man rührt 1 Tag bei Raumtemperatur, engt im Vakuum ein und destilliert den Rückstand im Hochvakuum.

Ausbeute: 840,7 g (84% der Theorie) gelbliches Öl
Kp.: 150-152 °C (4 mbar)

Beispiel II

2-Amino-3-ethoxycarbonyl-4-(4-fluorphenyl)-6-isopropyl-5-methoxycarbonyl-1,4-dihydropyridin

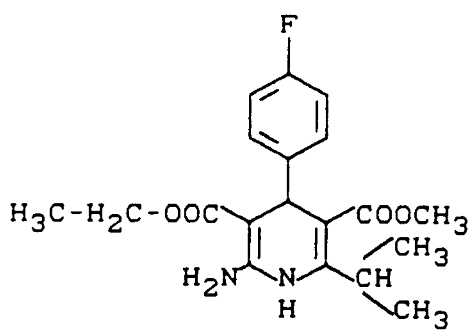

66,6 g (0,4 mol) 3,3-Diaminoacrylsäureethylester Hydrochlorid und 100 g (0,4 mol) der Verbindung aus Beispiel I werden mit 44 ml (0,4 mol) N-Methylmorpholin in 800 ml Isopropanol über Nacht zum Rückfluß gekocht. Man engt im Vakuum ein und chromatographiert den Rückstand in einer Säule (∅ 20 cm) an 2 kg Kieselgel 230-400 mesh mit Petrolether/Essigester (2:1)

Ausbeute: 109,7 g (75,7% der Theorie) farbl. Kristalle
Fp.: 161 °C (aus Ether/Petrolether)

Beispiel III

2-Amino-3-ethoxycarbonyl-4-(4-fluorphenyl)-6-isopropyl-5-methoxycarbonyl-pyridin

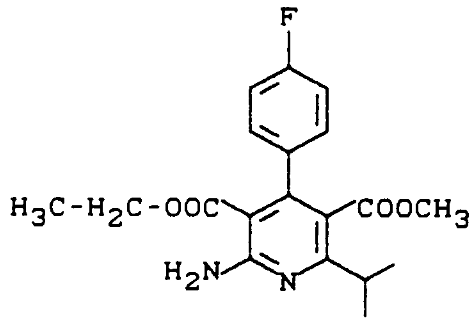

Zu einer Lösung von 36,2 g (0,1 mol) der Verbindung aus Beispiel II in 2 l Dichlormethan gibt man 22,7 g (0,1 mol) 2,3-Dichlor-4,5-dicyano-benzochinon und rührt 40 min bei Raumtemperatur. Man filtiert die Suspension in einer Glasnutsche über 1,5 kg Kieselgel 230 - 400 mesh und wäscht mit einem Gemisch aus Petrolether/Essigester 2:1 nach Das Eluat wird im Vakuum eingeengt und der verbleibende Rückstand in Ether/Petrolether ausgerührt und abgesaugt.

Ausbeute: 31,6 g (88% der Theorie)
Fp.: 141 °C

Beispiel IV

2-Amino-4-(4-fluorphenyl)-3-hydroxymethyl-6-isopropyl-5-methoxycarbonyl-pyridin

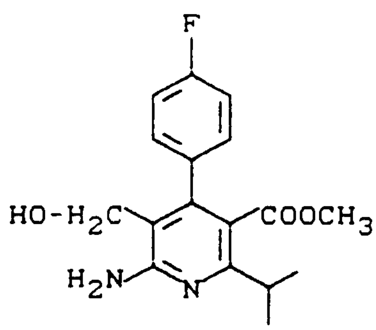

Unter Argon werden 100 ml (0,35 mol) einer 3,5 M Lösung von Natrium-bis-(2-methoxy-ethoxy)-dihydroaluminat in Toluol in 100 ml Tetrahydrofuran p.a. vorgelegt. Man tropft 63 g (175 mmol) der Verbindung aus Beispiel III in 700 ml Tetrahydrofuran gelöst zu und rührt 1 h bei 30 °C nach. Es werden vorsichtig 2 l Wasser zugetropft. Man trennt die Phasen und wäscht die wässrige Phase 2 mal mit 700 ml Essigester. Die vereinigten organischen Phasen werden mit 500 ml gesättigter Natriumchloridlösung gewaschen und mit Natriumsulfat getrocknet. Man filtriert und engt die Lösung im Vakuum ein, Der Rückstand wird in einer Säule (Ø 6 cm) an 400 g Kieselgel 230 - 400 mesh mit Petrolether/Essigester (1:1) chromatographiert. Man engt das Eluat im Vakuum ein und rührt den Rückstand in Ether/Petrolether aus,

Ausbeute: 45,2 g (81,2% der Theorie) farblose Kirstalle
Fp.: 137 °C

Beispiel V

5-(4-Fluorphenyl)-7-isopropyl-6-methoxycarbonyl-2-oxo-1,4-dihydro-2H-pyrido-[2,3-d][1,3]oxazin

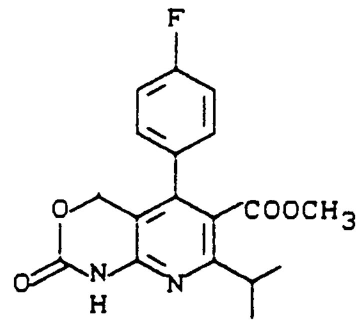

41,3 g (0,13 mol) der Verbindung aus Beispiel IV werden in 325 ml Toluol gelöst und mit 33,4 g (156 mmol) Diphenylcarbonat und 21,7 g (143 mmol) 1,8-Diazabicyclo-(5.4.0)-undec-7-en 30 min zum Rückfluß gekocht. Man kühlt ab und versetzt mit 300 ml Wasser und 150 ml Essigester. Man extrahiert die wässrige Phase mit 300 ml Essigester und wäscht die vereinigten organischen Phasen 2 mal mit 200 ml 0,1 M NaOH und 1 mal mit 200 ml gesättigter Natriumchloridlösung, trocknet mit Natriumsulfat und engt im Vakuum ein. Der verbleibenden Rückstand wird in einer Säule (Ø 20 cm) an 1,6 kg Kieselgel 230 - 400 mesh mit einem Gemisch aus Petrolether/Essigester (3:1) bis (1:1) chromatographiert.

Ausbeute: 16,6 g (37,1% der Theorie) farbl. Kristalle
Fp.: 175 °C

Beispiel VI

5-(4-Fluorphenyl)-7-isopropyl-6-hydroxymethyl-2-oxo-1,4-dihydro-2H-pyrido[2,3-d][1,3]oxazin

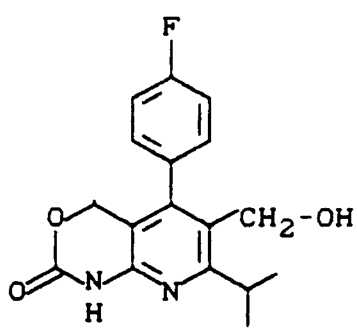

Unter Argon gibt man zu einer Lösung von 17,2 g (50 mmol) der Verbindung aus Beispiel V in 700 ml Toluol bei -75 °C langsam 100 ml einer 1,5 M Lösung von Diisobutylaluminiumhydrid in Toluol. Nach 1 h gibt man bei derselben Temperatur 33 ml 1,5 M Diisobutylaluminiumhydrid-Lösung zu, rührt eine weitere Stunde und gibt wieder 33 ml 1,5 M Diisobutylaluminiumhydrid-Lösung zu. Man rührt 2 h bei -75 °C und läßt dann auf Raumtemperatur erwärmen, wobei ab -30 °C vorsichtig mit 250 ml Wasser und 250 ml Essigester versetzt wird. Man saugt über Kieselgur ab und wäscht mit Essigester nach. Nach Phasentrennung extrahiert man die wäßrige Phase mit Essigester, wäscht die vereinigten organischen Phasen mit gesättigter Natriumchlorid-Lösung, trocknet über Natriumsulfat und engt im Vakuum ein. Der verbleibende Rückstand wird in Dichlormethan ausgerührt und abgesaugt. Man erhält 2,2 g (13,9 % der Theorie) der Titelverbindung. Die Mutterlauge chromatographiert man in einer Säule (Ø 6 cm) an 400 g Kieselgel 230-400 mesh mit einem Gradienten aus Petrolether/Essigester (2:1 bis 1:1). Man erhält 1,1 g (6,9% der Theorie) der Titelverbindung. Der Kieselgurrückstand wird 2 mal mit 300 ml Essigester ausgekocht, abgesaugt und das Filtrat im Vakuum eingeengt. Man erhält 8,7 g (55% der Theorie) der Titelverbindung.

Ausbeute: 12,0 g (75,9% der Theorie) farbl. Kristalle

Fp.: 154 ° C

Beispiel VII

5-(4-Fluorphenyl)-6-formyl-7-isopropyl-2-oxo-1,4-dihydro-2H-pyrido[2,3-d][1,3]oxazin

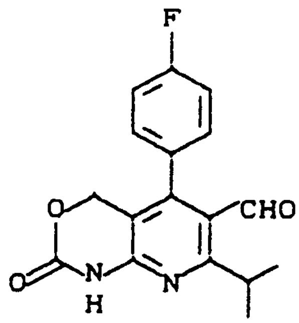

12 g (38 mmol) der Verbindung aus Beispiel VI werden in 1,4 l Dichlormethan suspendiert, mit 7,8 g neutralem Aluminiumoxid und 16,3 g (76 mmol) Pyridiniumchlorochromat versetzt und 1 h bei Raumtemperatur gerührt. Man gibt die Suspension auf eine Glasfritte (Porösität 3), die 600 g Kieselgel 230-400 mesh enthält, und eluiert ohne Trocken zu saugen mit Petrolether/Essigester 2:1. Das Eluat wird im Vakuum zur Trockene eingeengt und der verbleibende Rückstand in Ether/Petrolether ausgerührt.

Ausbeute: 10,8 g (90,5% der Theorie)
Fp.: 157 ° C

Beispiel VIII

5-(4-Fluorphenyl)-6-formyl-7-isopropyl-1-(1-picolyl)-2-oxo-1,4-dioxo-2H-pyrido[2,3-d][1,3]oxazin

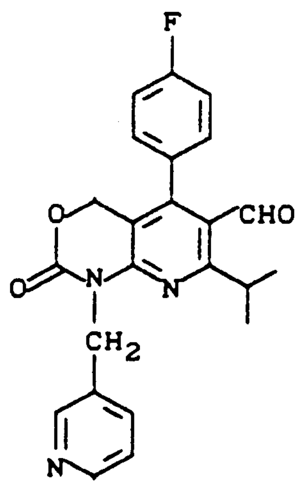

2,36 g (7,5 mmol) der Verbindung aus Beispiel VII werden in 60 ml wasserfreiem Dimethylformamid gelöst und mit 1,77 g (15,8 mmol) Kalium-tert.butylat versetzt. Man rührt 15 min bei Raumtemperatur und gibt 1,36 g (8,25 mmol) 3-Picolylchlorid Hydrochlorid zu. Es wird 100 min bei 60 ° C gerührt und anschließend auf 200 ml Eiswasser gegossen. Man extrahiert zweimal mit Essigester, wäscht die vereinigten organischen Phasen mit gesättigter Natriumchloridlösung, trocknet über Natriumsulfat und engt im Vakuum ein. Der verbleibende Rückstand wird in einer Säule (Ø 4 cm) an 100 g Kieselgel 230-400 mesh mit einem Gemisch aus Petrolether/Essigester 2:1 bis 1:1 chromatographiert.

Ausbeute: 1,0 g (32% der Theorie) farblose Kristalle
Fp.: 124 ° C (aus Ether)

Beispiel IX

5-(4-Fluorphenyl)-6-formyl-7-isopropyl-1-methyl-2-oxo-1,4-dihydro-2H-pyrido[2,3-d][1,3]oxazin

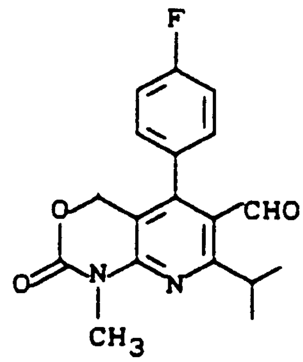

0,3 g (10 mmol) 80%iges Natriumhydrid werden unter leichtem Argonstrom in 12 ml wasserfreiem Tetrahydrofuran suspendiert, und mit 2,5 g (8 mmol) der Verbindung aus Beispiel VII in 20 ml Tetrahydrofuran versetzt. Man rührt 15 min bei Raumtemperatur und tropft 1,5 ml (24 mmol) Methyliodid zu. Es wird 90 min bei 40 bis 50 °C gerührt, abgekühlt, vorsichtig mit Wasser versetzt und zweimal mit Essigester extrahiert. Die vereinigten organischen Phasen werden mit gesättigter Natriumchloridlösung gewaschen, über Natriumsulfat getrocknet und im Vakuum zur Trockene eingeengt. Der verbleibende Rückstand wird in Ether/Petrolether ausgerührt und abgesaugt.

Ausbeute: 1,98 g (75% der Theorie) farbl. Kristalle

Fp.: 147 °C

Beispiel X

1-Benzyl-5-(4-fluorphenyl)-6-formyl-7-isopropyl-2-oxo-1,4-dihydro-2H-pyrido[2,3-d][1,3]oxazin

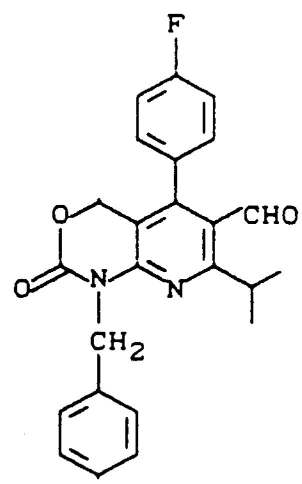

2,36 g (7,5 mmol) der Verbindung aus Beispiel VII werden in 20 ml wasserfreiem Dimethylformamid gelöst, und unter leichtem Argonstrom mit 0,28 g (9,4 mmol) 80%igem Natriumhydrid versetzt. Es wird 15 min bei Raumtemperatur gerührt und mit 1,54 g (9 mmol) Benzylbromid versetzt. Nach 60 min gibt man vorsichtig 30 ml Wasser zu und extrahiert zweimal mit Essigester. Die vereinigten organischen Phasen werden mit gesättigter Natriumchlorid-Lösung gewaschen, über Natriumsulfat getrocknet und im Vakuum zur Trockene eingeengt. Der verbleibende Rückstand wird in einer Säule (Ø 3 cm) an 50 g Kieselgel 230-400 mesh mit Petrolether/Essigester 4:1 chromatographiert.

Ausbeute: 2,3 g (75% der Theorie) farbl. amorpher Feststoff

$R_f$ = 0,2 (Petrolether / Essigester 5:1)

Beispiel XI

1-Allyl-5-(4-fluorphenyl)-6-formyl-7-isopropyl-2-oxo-1,4-dihydro-2H-pyrido[2,3][1,3]oxazin

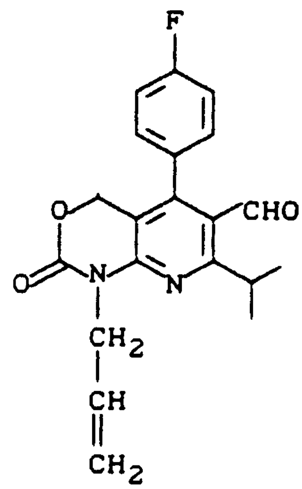

In Analogie zu Beispiel X erhält man mit 1,1 g (9 mmol) 3-Brompropen 1,55 g (58% der Theorie) der Titelverbindung.

Fp.: 92 °C (aus Ether/Petrolether)

Beispiel XII-a

(E)-3-[5-(4-Fluorphenyl)-7-isopropyl-2-oxo-1,4-dihydro-2H-pyrido[2,3-d][1,3]oxazin-6-yl]prop-2-enal

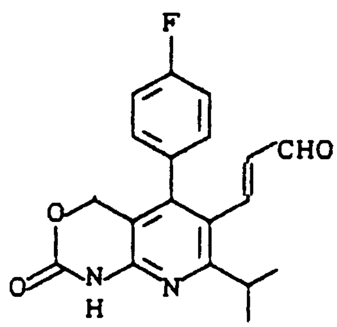

Zu einer Suspension von 0,58 g (19,2 mmol) 80%igem Natriumhydrid in 24 ml wasserfreiem Tetrahydrofuran tropft man innerhalb von 10 min bei 0 - 5 °C unter Argon eine Lösung von 2,5 g (9,6 mmol) Diethyl-2-(cyclohexylamino)-vinyl-phosphonat in 24 ml Tetrahydrofuran. Es wird 15 min bei 0 °C gerührt, innerhalb von 20 min eine Lösung von 2,5 g (8 mmol) der Verbindung aus Beispiel VII in 24 ml Tetrahydrofuran bei 0-5 °C zugetropft, 30 min bei Raumtemperatur und 2 h unter Rückfluß gerührt. Man kühlt ab, versetzt vorsichtig mit 75 ml Wasser, extrahiert zweimal mit Essigester, wäscht die vereinigten organischen Phasen mit gesättigter Natriumchloridlösung und engt im Vakuum ein, Der Rückstand wird mit einer Mischung aus 100 ml Toluol, 100 ml Wasser und 5,3 g (41,6 mmol) Oxalsäuredihydrat 1 h zum Rückfluß gekocht. Man kühlt ab, gibt 100 ml Essigester zu und trennt die Phasen. Man extrahiert die wässrige Phase 2 mal mit Essigester, wäscht die vereinigten organischen Phasen mit gesättigter Natriumchloridlösung, trocknet über Natriumsulfat und engt im Vakuum ein. Der Rückstand wird in einer Säule (Ø 4 cm) mit 100 g Kieselgel 230-400 mesh mit einem Gemisch aus Petrolether/Essigester 2:1 bis Essigester chromatographiert.

Ausbeute: 0,85 g (31% der Theorie)

Fp.: 223 °C

Die in Tabelle 1 aufgeführten Beispiele wurden in Analogie zur Vorschrift für Beispiel XII-a unter Verwendung von 1,2 Moläquivalent Natriumhydrid hergestellt:

Tabelle 1

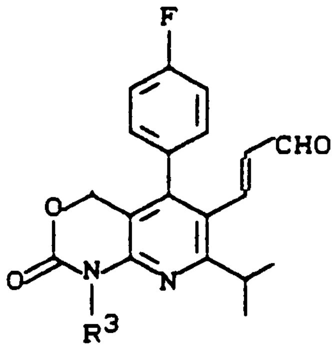

| Bsp. Nr. | R | Ausgangsmaterial Beispiel | Ausbeute | Fp. (°C) |
|---|---|---|---|---|
| XII-b | $-CH_3$ | IX | 60% | 122° C |
| XII-c | $-CH_2-$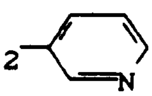 | VIII | 38% | gelbl. amorph. |
| XII-d | $-CH_2-$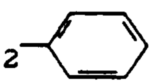 | X | 54% | 179° C |
| XII-e | $-CH_2-CH=CH_2$ | XI | 42% | 97° C |

Herstellungsbeispiele (allgemeine Formel I)

Beispiel 1-a

Methyl-erythro-(E)-7-[5-(4-fluorphenyl)-7-isopropyl-2-oxo-1,4-dihydro-2H-pyrido[2,3-d][1,3]oxazin-6-yl]-3,5-dihydroxy-hept-6-enoat

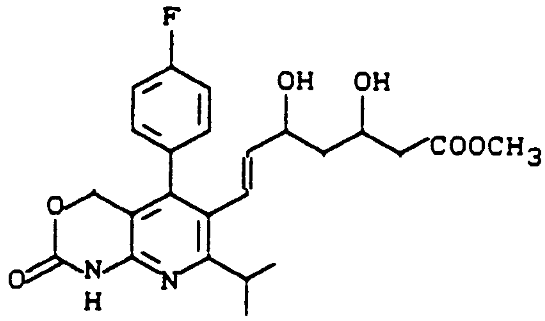

Zu einer Suspension von 0,23 g (7,5 mmol) 80%igem Natriumhydrid in 10 ml wasserfreiem Tetrahydrofuran gibt man bei 0 - 5 °C tropfenweise 0,44 g (3,75 mmol) Acetessigsäuremethylester. Nach 15 min tropft man innerhalb von 10 min 4,7 ml (7,785 mmol) 15%iges Butyllithium in Hexan zu und hält weitere 15 min bei 0 - 5 °C. Dann werden 0,85 g (2,5 mmol) der Verbindung aus Beispiel XII-a in 10 ml Tetrahydrofuran zugegeben und 30 min bei Raumtemperatur gerührt. Anschließend versetzt man vorsichtig mit 0,96 g (16 mmol) Essigsäure in 25 ml Wasser, extrahiert dreimal mit Essigester, wäscht die vereinigten organischen Phasen mit gesättigter Natriumchloridlösung, trocknet über Natriumsulfat und engt im Vakuum ein. Der verbleibende Rückstand wird in 15 ml wasserfreiem Tetrahydrofuran gelöst. Man gibt 3 ml einer 1 M Triethylboranlösung in Tetrahydrofuran zu und bläst 5 min Luft durch die Lösung. Bei -78 °C gibt man 114 mg (3 mmol) Natriumborhydrid zu. Dann tropft man 2,5 ml Methanol zu und hält 1 h bei -78 °C bis -75 °C, Man läßt auf Raumtemperatur erwärmen, wobei ab -30 °C 8,3 ml 30%iges Wasserstoffperoxid und 30 ml Wasser zugesetzt werden. Es wird zweimal mit Essigester extrahiert, die vereinigten organischen Phasen mit gesättigter Natriumchloridlösung gewaschen, über Natriumsulfat getrocknet und im Vakuum eingeengt. Der verbleibende Rückstand wird in einer Säule (Ø 3 cm) an 30 g Kieselgel 230-400 mesh mit Petrolether/Essigester (1:1) chromatographiert.

Ausbeute: 0,25 g (21% der Theorie) amorpher Feststoff

$R_f$ = 0,28 (Essigester / Petrolether 2:1)

$^1$H-NMR ($CDCl_3$) $\delta$ = 7,65 (b, 1H); 7,2 - 7,0 (m, 4H); 6,35 (d, 1H); 5,3 (dd, 1H); 5,0 (s, 2H); 4,35 (m, 1H); 4,1 (m, 1H); 3,75 (s, 3H); 3,65 (b, 1H); 3,4 - 3,2 (m, 2H); 2,55 - 2,45 (m, 2H); 1,5 - 1,3 (m, 2H); 1,25 (d, 6H).

In Analogie zu Beispiel 1-a wurden die in Tabelle 2 aufgeführten Beispiele hergestellt, wobei 2 Moläquivalente Natriumhydrid, 2,1 Butyllithium und 4,4 Essigsäure verwendet wurden.

Tabelle 2

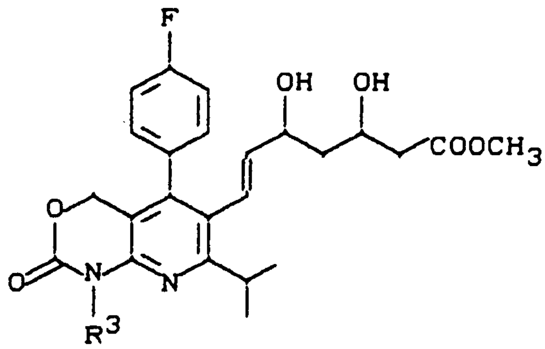

| Bsp. Nr. | $R^3$ | hergestellt aus Beispiel | Ausbeute | Fp. (°C) |
|---|---|---|---|---|
| 1-b | $-CH_3$ | XII-b | 30% | farbl. amorph. |
| 1-c | $-CH_2-$ 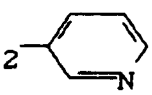 | XII-c | 3% | farbl. Öl |
| 1-d | $-CH_2-$ 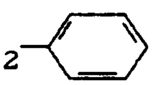 | XII-d | 15% | farbl. amorph. |
| 1-e | $-CH_2-CH=CH_2$ | XII-e | 12% | farbl. Öl |

Beispiel 2-b

Erythro-(E)-7-[5-(4-Fluorphenyl)-7-isopropyl-1-methyl-2-oxo-1,4-dihydro-2H-pyrido[2,3-d][1,3]oxazin-6-yl]-3,5-dihydroxy-hept-6-ensäure-Natriumsalz

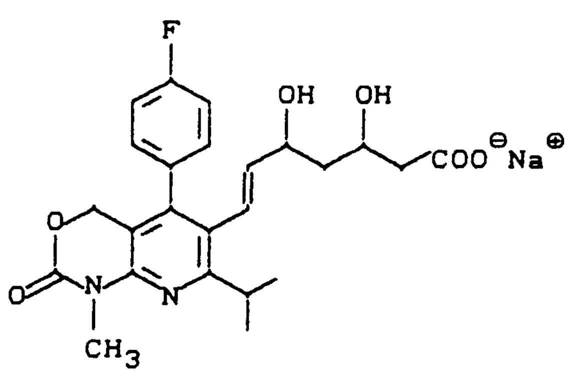

Zu einer Lösung von 142 mg (0,3 mmol) der Verbindung aus Beispiel 1-b in 3 ml Tetrahydrofuran gibt man 3 ml (0,3 mmol) 0,1 M Natronlauge zu und rührt 1 h bei Raumtemperatur, engt im Vakuum zur Trockene ein und trocknet im Hochvakuum über Phosphorpentoxid.

Ausbeute: 134 mg (93% der Theorie)

Fp.: 93 °C (Zers.)

In Analogie zur Vorschrift des Beispiels 1-b wurden die in Tabelle 3 aufgeführten Beispiele hergestellt:

Tabelle 3

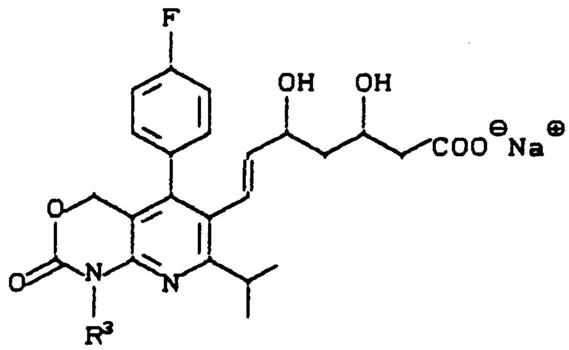

| Bsp. Nr. | $R^3$ | hergestellt aus Beispiel | Ausbeute | Fp. (°C) |
|---|---|---|---|---|
| 2-d | $-CH_2-$ 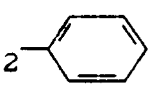 | 1-d | 71% | 123-125 (Zers.) |
| 2-e | $-CH_2-CH=CH_2$ | 1-e | 94% | 127-130 (Zers.) |

Beispiel 3-b

6-[5-(4-Fluorphenyl)-7-isopropyl-1-methyl-2-oxo-1,4-dihydro-2H-pyrido[2,3-d][1,3]-oxazin-6-yl]-3,5-dihydroxy-heptan-säuremethylester

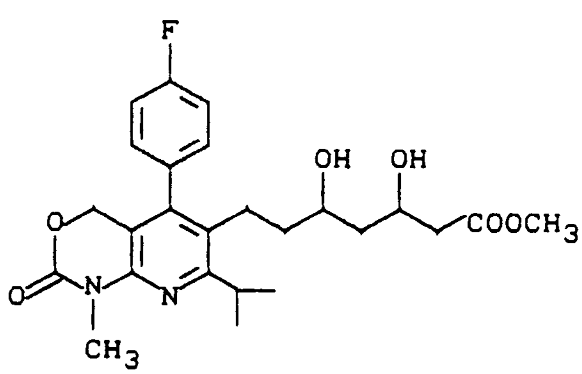

Eine Lösung von 153 mg (0,32 mmol) der Verbindung aus Beispiel 1-b in 25 ml Methanol und 20 µl Triethylamin werden über 120 mg 10% Palladium/Kohle 4 h bei Raumtemperatur und Normaldruck hydriert, Man filtriert den Katalysator ab, engt ein, löst den Rückstand in Essigester und wäscht mit gesättigter Kochsalzlösung. Die organische Phase wird getrocknet und zur Trockene eingeengt.

Ausbeute: 123 mg (80% der Theorie) farbloses Öl.

$^1$H-NMR ($CDCl_3$): δ = 1,15 - 1,5 (m, 10H), 2,3 - 2,55 (m, 4H); 3,2 (m, 1H); 3,3 (b, 1H); 3,45 (s, 3H); 3,6 (m, 2H); 3,65 (s, 3H); 4,1 (m, 1H); 4,7 (m, 2H); 7,0 - 7,15 (m, 4H).

**Patentansprüche**

**Patentansprüche für folgende Vertragsstaaten : AT, BE, CH, DE, DK, FR, GB, GR, IT, LI, LU, NL, SE**

**1.** Substituierte Pyrido-oxazine der allgemeinen Formel

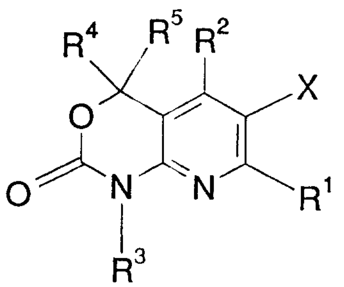

(I)

in welcher

$R^1$
- für geradkettiges oder verzweigtes Alkyl mit bis zu 6 Kohlenstoffatomen steht,
- für Cyclopropyl, Cyclopentyl oder Cyclohexyl steht,

$R^2$
- für Phenyl steht, das gegebenenfall durch geradkettiges oder verzweigtes Alkyl mit bis zu 4 Kohlenstoffatomen, Trifluormethyl, Fluor, Chlor oder Brom substituiert ist,

$R^3$
- für Wasserstoff steht oder
- für geradkettiges oder verzweigtes Alkyl mit bis zu 8 Kohlenstoffatomen steht, das gegebenenfalls durch Cyano, Alkoxy mit bis zu 4 Kohlenstoffatomen, Phenyl oder Pyridyl substituiert ist,
- für geradkettiges oder verzweigtes Alkenyl mit bis zu 6 Kohlenstoffatomen seth,
- für Cyclopropyl, Cyclopentyl oder Cyclohexyl steht,

$R^4$ und $R^5$ für Wasserstoff stehen,

X
- für einen Rest der Formel -A-B steht, worin

A
- eine Gruppe der Formel $-CH_2-CH_2-$ oder $-CH=CH-$bedeutet,

B
- eine Gruppe der Formel

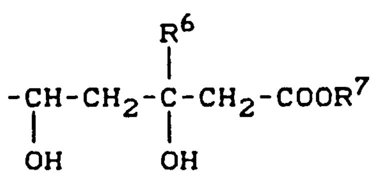

oder

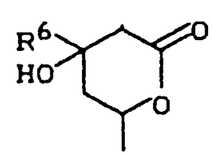

bedeutet, worin

$R^6$ - Wasserstoff bedeutet und

$R^7$ - Wasserstoff oder geradkettiges oder verzweigtes Alkyl mit bis zu 8 Kohlenstoffatomen oder Benzyl bedeutet, oder
- Phenyl oder ein Kation bedeutet

und deren Salze.

**2.** Substituierte Pyrido-oxazine der allgemeinen Formel nach Anspruch 1, in welcher

$R^1$ - für geradkettiges oder verzweigtes Alkyl mit bis zu 4 Kohlenstoffatomen oder Cyclopropyl steht,

$R^2$ - für Phenyl steht, das gegebenenfalls durch Methyl, Trifluormethyl, Fluor oder Chlor substituiert ist,

$R^3$
- für Wasserstoff steht oder
- für geradkettiges oder verzweigtes Alkyl mit bis zu 6 Kohlenstoffatomen steht, das gegebenenfalls durch Cyano, Phenyl oder Pyridyl substituiert ist,
- für geradkettiges oder verzweigtes Alkenyl mit bis zu 4 Kohlenstoffatomen steht,

$R^4$ und $R^5$ für Wasserstoff stehen,

X - für einen Rest der Formel -A-B steht, worin

A - die -CH=CH- oder $CH_2$-$CH_2$-Gruppe bedeutet,

B - eine Gruppe der Formel

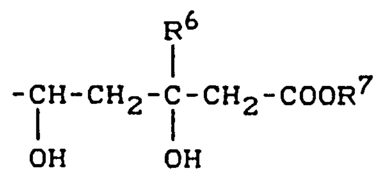

oder

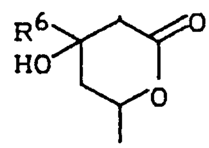

bedeutet, worin

$R^6$ Wasserstoff bedeutet, und

$R^7$ Wasserstoff, Methyl, Ethyl, Propyl, Isopropyl, Butyl, Isobutyl, tert.-Butyl oder Benzyl bedeutet, oder ein Natrium-, Kalium-, Caclium-, Magnesium- oder Ammoniumion bedeutet,

und deren Salze.

**3.** Substituierte Pyrido-oxazine nach den Ansprüchen 1 und 2 zur Bekämpfung von Krankheiten.

**4.** Verfahren zur Herstellung von substituierten Pyrido-oxazinen und deren Salzen nach Ansprüchen 1 und 2, dadurch gekennzeichnet, daß man Ketone der allgemeinen Formel (VIII)

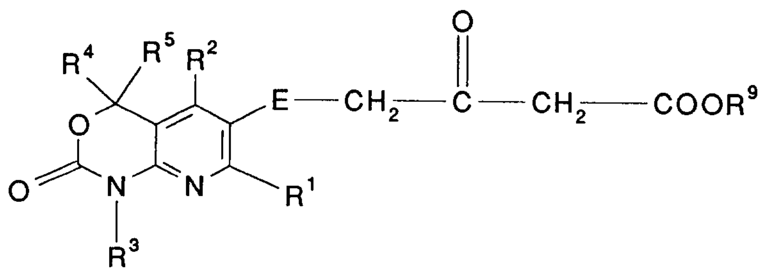

(VIII)

in welcher
$R^1$, $R^2$, $R^3$, $R^4$ und $R^5$ die oben angegebene Bedeutung haben,
$R^9$ für Alkyl steht, und
E für eine Gruppe

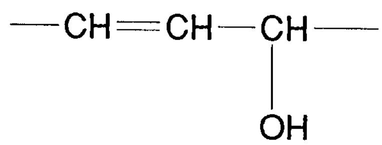

steht,
reduziert,
im Fall der Herstellung der Lactone die Carbonsäuren cyclisiert,
im Fall der Herstellung der Ethylenverbindungen (A = $-CH_2.-CH_2-$) die Ethenverbindungen (A = -CH = CH-) nach üblichen Methoden hydriert,
und gegebenenfalls Isomeren trennt.

5. Arzneimittel enthaltend mindestens ein substituiertes Pyrido-oxazin nach den Ansprüchen 1 und 2.

6. Verfahren zur Herstellung eines Arzneimittels nach Anspruch 5, dadurch gekennzeichnet, daß man das oder die Pyrido-oxazine gegebenenfalls mit Hilfe üblicher Hilfs- und Trägerstoff in eine geeignete Applikationsform bringt.

7. Verwendung der substituierten Pyrido-oxazine nach den Ansprüchen 1 und 2 zur Herstellung von Arzneimitteln.

8. Verwendung der substituierten Pyrido-oxazine nach den Ansprüchen 1 und 2 zur Herstellung von Arzneimitteln zur Behandlung von Hyperlipoproteinämie, Lipoproteinämie und Arteriosklerose.

**Patentanspruch für folgenden Vertragsstaat : ES**

1. Verfahren zur Herstellung von substituierten Pyrido-oxazinen der allgemeinen Formel

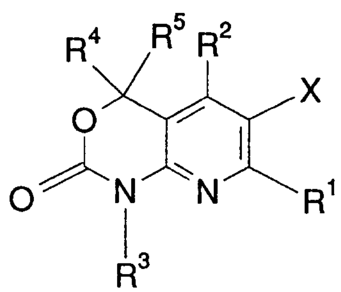

(I)

in welcher

$R^1$
- für geradkettiges oder verzweigtes Alkyl mit bis zu 6 Kohlenstoffatomen steht,
- für Cyclopropyl, Cyclopentyl oder Cyclohexyl steht,

$R^2$
- für Phenyl steht, das gegebenenfall durch geradkettiges oder verzweigtes Alkyl mit bis zu 4 Kohlenstoffatomen, Trifluormethyl, Fluor, Chlor oder Brom substituiert ist,

$R^3$
- für Wasserstoff steht oder
- für geradkettiges oder verzweigtes Alkyl mit bis zu 8 Kohlenstoffatomen steht, das gegebenenfalls durch Cyano, Alkoxy mit bis zu 4 Kohlenstoffatomen, Phenyl oder Pyridyl substituiert ist,
- für geradkettiges oder verzweigtes Alkenyl mit bis zu 6 Kohlenstoffatomen seht,
- für Cyclopropyl, Cyclopentyl oder Cyclohexyl steht,

$R^4$ und $R^5$ für Wasserstoff stehen,

X
- für einen Rest der Formel -A-B steht,
  worin

A
- eine Gruppe der Formel $-CH_2-CH_2-$ oder $-CH=CH-$bedeutet,

B
- eine Gruppe der Formel

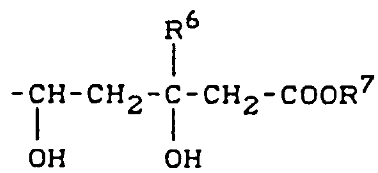

oder

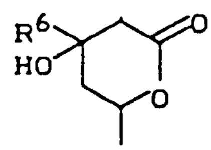

bedeutet,
worin

$R^6$
- Wasserstoff bedeutet
  und

$R^7$
- Wasserstoff oder geradkettiges oder verzweigtes Alkyl mit bis zu 8 Kohlenstoffatomen oder Benzyl bedeutet, oder
- Phenyl oder ein Kation bedeutet

und deren Salze,

dadurch gekennzeichnet, daß man Ketone der allgemeinen Formel (VIII)

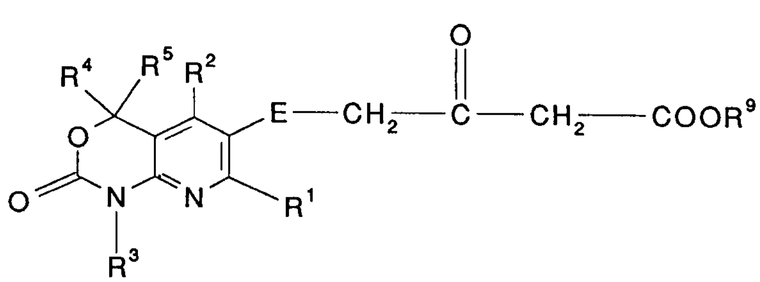

(VIII)

in welcher

$R^1$, $R^2$, $R^3$ $R^4$ und $R^5$ die oben angegebene Bedeutung haben,

$R^9$ für Alkyl steht, und

E für eine Gruppe

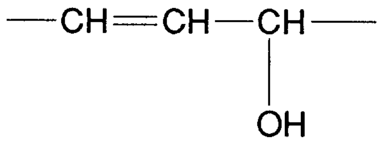

steht,
reduziert,
im Fall der Herstellung der Lactone die Carbonsäuren cyclisiert,
im Fall der Herstellung der Ethylenverbindungen (A = $-CH_2.-CH_2-$) die Ethenverbindungen (A = -CH = CH-) nach üblichen Methoden hydriert,
und gegebenenfalls Isomeren trennt.

## Claims

**Claims for the following Contracting States : AT, BE, CH, DE, DK, FR, GB, GR, IT, LI, LU, NL, SE**

**1.** Substituted pyrido-oxazines of the general formula

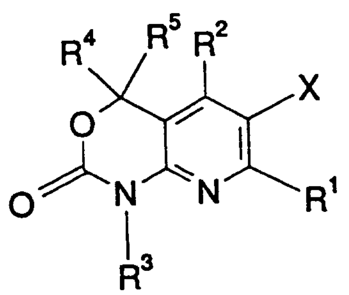

(I)

in which

$R^1$
- represents straight-chain or branched alkyl having up to 6 carbon atoms,
- represents cyclopropyl, cyclopentyl or cyclohexyl,

$R^2$
- represents phenyl which is optionally substituted by straight-chain or branched alkyl having up to 4 carbon atoms, trifluoromethyl, fluorine, chlorine or bromine,

$R^3$
- represents hydrogen or
- represents straight-chain or branched alkyl having up to 8 carbon atoms, which is optionally substituted by cyano, alkoxy having up to 4 carbon atoms, phenyl or pyridyl,
- represents straight-chain or branched alkenyl having up to 6 carbon atoms,
- represents cyclopropyl, cyclopentyl or cyclohexyl,

$R^4$ and $R^5$ represent hydrogen,

X
- represents a radical of the formula -A-B, in which

A
- denotes a group of the formula $-CH_2-CH_2-$ or -CH = CH-,

B

- denotes a group of the formula

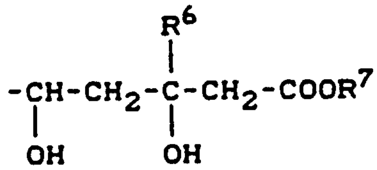

or

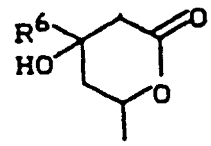

in which

$R^6$
- denotes hydrogen and

$R^7$
- denotes hydrogen or straight-chain or branched alkyl having up to 8 carbon atoms or benzyl, or denotes phenyl or a cation,

and their salts.

**2.** Substituted pyrido-oxazines of the general formula according to Claim 1 in which

$R^1$
- represents straight-chain or branched alkyl having up to 4 carbon atoms or cyclopropyl,

$R^2$
- represents phenyl which is optionally substituted by methyl, trifluoromethyl, fluorine or chlorine,

$R^3$
- represents hydrogen or
- represents straight-chain or branched alkyl having up to 6 carbon atoms, which is optionally substituted by cyano, phenyl or pyridyl,
- represents straight-chain or branched alkenyl having up to 4 carbon atoms,

$R^4$ and $R^5$ represent hydrogen,

X
- represents a radical of the formula -A-B, in which

A
- denotes the $-CH=CH-$ or $-CH_2-CH_2-$group,

B
- denotes a group of the formula

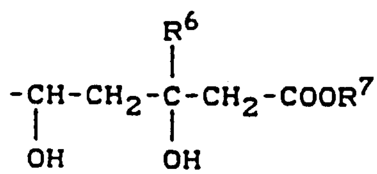

or

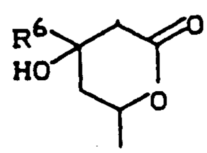

in which

$R^6$ denotes hydrogen, and

$R^7$ denotes hydrogen, methyl, ethyl, propyl, isopropyl, butyl, isobutyl, tert-butyl or benzyl, or denotes a sodium, potassium, calcium, magnesium or ammonium ion,

and their salts.

3. Substituted pyrido-oxazines according to Claims 1 and 2 for the control of diseases.

4. Process for the preparation of substituted pyrido-oxazines and their salts according to Claims 1 and 2, characterised in that ketones of the general formula (VIII)

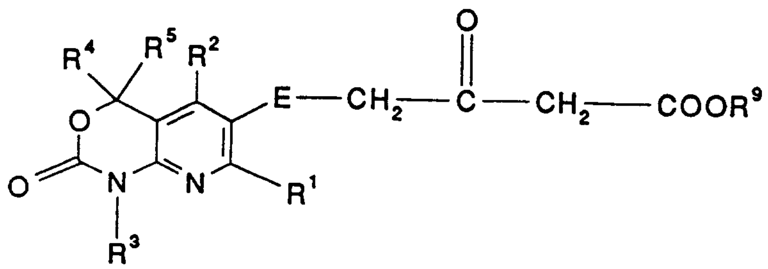

(VIII)

in which
$R^1$, $R^2$, $R^3$, $R^4$ and $R^5$ have the abovementioned meaning,
$R^9$ represents alkyl, and
E represents a group

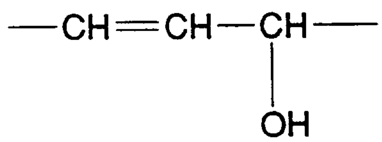

are reduced,
in the case of the preparation of the lactones, the carboxylic acids are cyclised,
in the case of the preparation of the ethylene compounds (A = $-CH_2-CH_2-$), the ethene compounds (A = $-CH=CH-$) are hydrogenated by customary methods,
and, if appropriate, isomers are separated.

5. Medicaments containing at least one substituted pyrido-oxazine according to Claims 1 and 2.

6. Process for the production of a medicament according to Claim 5, characterised in that the pyrido-oxazine(s) are brought into a suitable administration form, if appropriate, with the aid of customary auxiliaries and excipients.

7. Use of the substituted pyrido-oxazines according to Claims 1 and 2 for the production of medicaments.

8. Use of the substituted pyrido-oxazines according to Claims 1 and 2 for the production of medicaments for the treatment of hyperlipoproteinaemia, lipoproteinaemia and arteriosclerosis.

**Claim for the following Contracting State : ES**

**1.** Process for the preparation of substituted pyrido-oxazines of the general formula

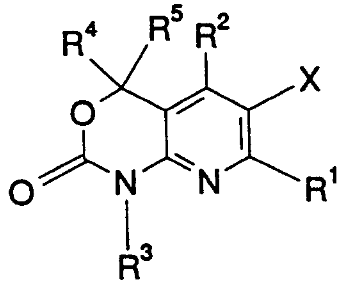

(I)

in which

$R^1$
- represents straight-chain or branched alkyl having up to 6 carbon atoms,
- represents cyclopropyl, cyclopentyl or cyclohexyl,

$R^2$
- represents phenyl which is optionally substituted by straight-chain or branched alkyl having up to 4 carbon atoms, trifluoromethyl, fluorine, chlorine or bromine,

$R^3$
- represents hydrogen or
- represents straight-chain or branched alkyl having up to 8 carbon atoms, which is optionally substituted by cyano, alkoxy having up to 4 carbon atoms, phenyl or pyridyl,
- represents straight-chain or branched alkenyl having up to 6 carbon atoms,
- represents cyclopropyl, cyclopentyl or cyclohexyl,

$R^4$ and $R^5$ represent hydrogen,

X
- represents a radical of the formula -A-B, in which

A
- denotes a group of the formula $-CH_2-CH_2-$ or $-CH=CH-$,

B
- denotes a group of the formula

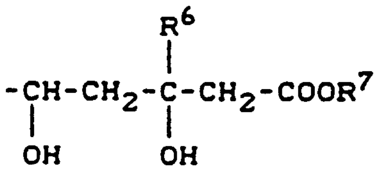

or

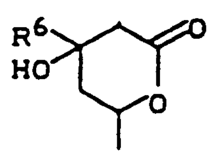

in which

$R^6$
- denotes hydrogen and

$R^7$
- denotes hydrogen or straight-chain or branched alkyl having up to 8 carbon atoms or benzyl, or
- denotes phenyl or a cation,

and their salts,

characterised in that ketones of the general formula (VIII)

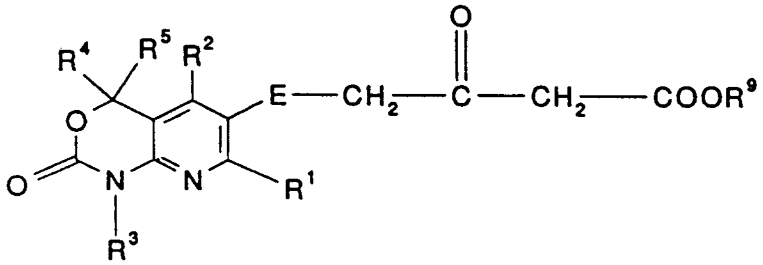

(VIII)

in which
$R^1$, $R^2$, $R^3$, $R^4$ and $R^5$ have the abovementioned meaning,
$R^9$ represents alkyl, and
E represents a group

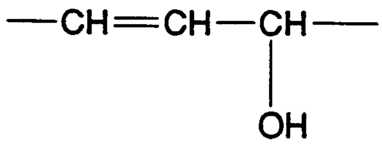

are reduced,
in the case of the preparation of the lactones, the carboxylic acids are cyclised,
in the case of the preparation of the ethylene compounds (A = $-CH_2-CH_2-$), the ethene compounds (A = $-CH=CH-$) are hydrogenated by customary methods,
and, if appropriate, isomers are separated.

## Revendications

**Revendications pour les Etats contractants suivants : AT, BE, CH, DE, DK, FR, GB, GR, IT, LI, LU, NL, SE**

**1.** Pyrido-oxazines substituées répondant à la formule générale

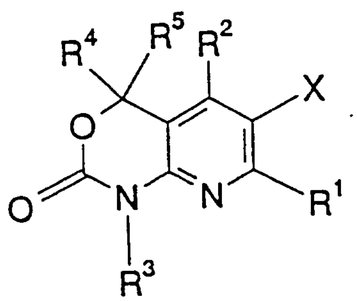

(I)

dans laquelle

$R^1$ représente un groupe alkyle à chaîne droite ou ramifiée contenant jusqu'à 6 atomes de carbone, un groupe cyclopropyle, un groupe cyclopentyle ou un groupe cyclohexyle,

$R^2$ représente un groupe phényle qui porte éventuellement un ou plusieurs substituants identiques ou différents alkyle à chaîne droite ou ramifiée contenant jusqu'à 4 atomes de carbone, trifluorométhyle, fluoro, chloro ou bromo,

$R^3$ représente un atome d'hydrogène ou
un groupe alkyle à chaîne droite ou ramifiée contenant jusqu'à 8 atomes de carbone, qui porte éventuellement un ou plusieurs substituants identiques ou différents cyano, alcoxy contenant jusqu'à 4 atomes de carbone, phényle ou pyridyle,
un groupe alcényle à chaîne droite ou ramifiée contenant jusqu'à 6 atomes de carbone,

un groupe cyclopropyle, un groupe cyclopentyle ou un groupe cyclohexyle,

$R^4$ et $R^5$ représentent un atome d'hydrogène,

X représente un radical de formule -A-B dans laquelle

A représente un groupe de formule $-CH_2-CH_2-$ ou $-CH=CH-$,

B représente un groupe de formules

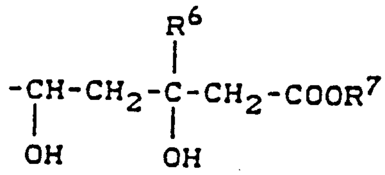

ou

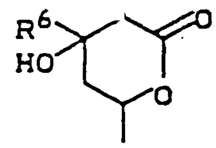

dans lesquelles

$R^6$ représente un atome d'hydrogène et

$R^7$ représente un atome d'hydrogène ou un groupe alkyle à chaîne droite ou ramifiée contenant jusqu'à 8 atomes de carbone, ou encore un groupe benzyle, ou bien représente un groupe phényle ou encore un cation,

ainsi que leurs sels.

**2.** Pyrido-oxazines substituées répondant à la formule générale selon la revendication 1, dans laquelle

$R^1$ représente un groupe alkyle à chaîne droite ou ramifiée contenant jusqu'à 4 atomes de carbone, ou encore un groupe cyclopropyle,

$R^2$ représente un groupe phényle qui porte éventuellement un ou plusieurs substituants identiques ou différents méthyle, trifluorométhyle, fluoro ou chloro,

$R^3$ représente un atome d'hydrogène ou un groupe alkyle à chaîne droite ou ramifiée contenant jusqu'à 6 atomes de carbone, qui porte éventuellement un ou plusieurs substituants identiques ou différents cyano, phényle ou pyridyle, un groupe alcényle à chaîne droite ou ramifiée contenant jusqu'à 4 atomes de carbone,

$R^4$ et $R^5$ représentent un atome d'hydrogène,

X représente un radical de formule -A-B dans laquelle

A représente le groupe $-CH_2-CH_2-$ ou $-CH=CH-$,

B représente un groupe répondant aux formules

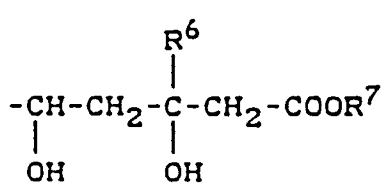

ou

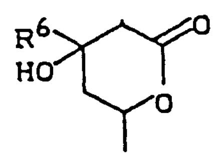

dans lesquelles

$R^6$ représente un atome d'hydrogène et

$R^7$ représente un atome d'hydrogène, un groupe méthyle, un groupe éthyle, un groupe propyle, un groupe isopropyle, un groupe butyle, un groupe isobutyle, un groupe tert.-butyle ou un groupe benzyle, ou encore un ion sodium, potassium, calcium, magnésium ou ammonium,

ainsi que leurs sels.

**3.** Pyrido-oxazines substituées selon les revendications 1 et 2, pour lutter contre des maladies.

**4.** Procédé pour la préparation de pyrido-oxazines substituées et de leurs sels selon les revendications 1 et 2, caractérisé en ce qu'on soumet à une réduction des cétones répondant à la formule générale (VIII)

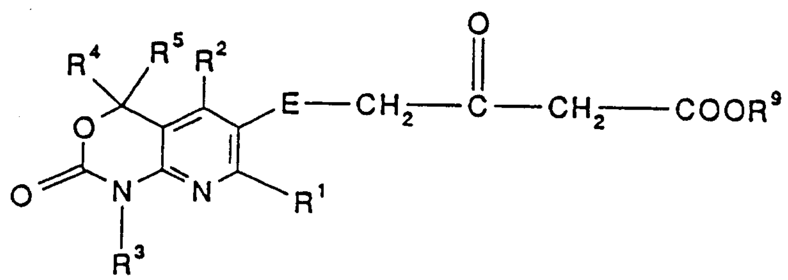

(VIII)

dans laquelle

| | |
|---|---|
| $R^1$, $R^2$, $R^3$, $R^4$ et $R^5$ | ont la signification indiquée ci-dessus, |
| $R^9$ | représente un groupe alkyle et |
| E | représente un groupe |

-CH=CH-CH-
|
OH

en cas de préparation de lactones, on cyclise les acides carboxyliques,
en cas de préparation des composés éthylène ($A = -CH_2-CH_2$), on soumet à une hydrogénation les composés d'éthène ($A = -CH = CH-$) conformément à des procédés habituels,
et éventuellement on sépare les isomères.

**5.** Médicament contenant au moins une pyrido-oxazine substituée selon les revendications 1 et 2.

**6.** Procédé pour la préparation d'un médicament selon la revendication 5, caractérisé en ce qu'on amène, dans une forme d'application appropriée, la ou les pyrido-oxazines éventuellement à l'aide de substances auxiliaires et de support habituelles.

**7.** Utilisation des pyrido-oxazines substituées selon les revendications 1 et 2, pour la préparation de médicaments.

**8.** Utilisation des pyrido-oxazines substituées selon les revendications 1 et 2, pour la préparation de médicaments pour traiter l'hyperlipoprotéinémie, la lipoprotéinémie et l'artériosclérose.

**Revendication pour l'Etat contractant suivant : ES**

1. Procédé pour la préparation de pyrido-oxazines substituées répondant à la formule générale

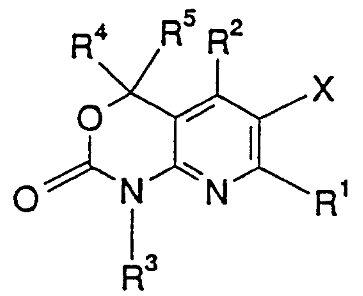

(I)

dans laquelle

| | |
|---|---|
| $R^1$ | représente un groupe alkyle à chaîne droite ou ramifiée contenant jusqu'à 6 atomes de carbone, un groupe cyclopropyle, un groupe cyclopentyle ou un groupe cyclohexyle, |
| $R^2$ | représente un groupe phényle qui porte éventuellement un ou plusieurs substituants identiques ou différents alkyle à chaîne droite ou ramifiée contenant jusqu'à 4 atomes de carbone, trifluorométhyle, fluoro, chloro ou bromo, |
| $R^3$ | représente un atome d'hydrogène ou<br>un groupe alkyle à chaîne droite ou ramifiée contenant jusqu'à 8 atomes de carbone, qui porte éventuellement un ou plusieurs substituants identiques ou différents cyano, alcoxy contenant jusqu'à 4 atomes de carbone, phényle ou pyridyle,<br>un groupe alcényle à chaîne droite ou ramifiée contenant jusqu'à 6 atomes de carbone, un groupe cyclopropyle, un groupe cyclopentyle ou un groupe cyclohexyle, |
| $R^4$ et $R^5$ | représentent un atome d'hydrogène, |
| X | représente un radical de formule -A-B<br>dans laquelle |
| A | représente le groupe de formule $-CH_2-CH_2-$ ou $-CH=CH-$, |
| B | représente un groupe répondant aux formules |

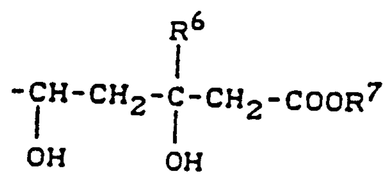

ou

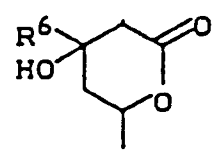

dans lesquelles

| | |
|---|---|
| $R^6$ | représente un atome d'hydrogène<br>et |
| $R^7$ | représente un atome d'hydrogène ou un groupe alkyle à chaîne droite ou ramifiée contenant jusqu'à 8 atomes de carbone, ou encore un groupe benzyle, ou bien<br>un groupe phényle ou encore un cation |

et de leurs sels, caractérisé en ce qu'on soumet à une réduction des cétones répondant à la formule générale (VIII)

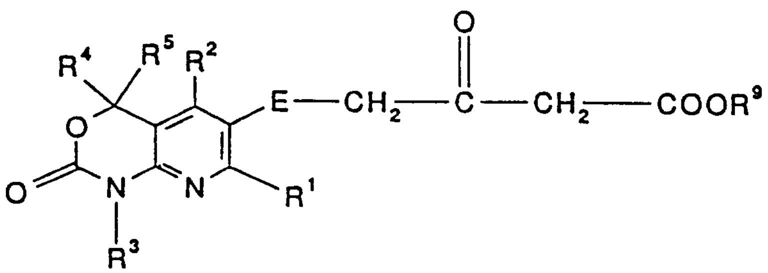 (VIII)

dans laquelle

| | |
|---|---|
| $R^1$, $R^2$, $R^3$, $R^4$ et $R^5$ | ont la signification indiquée ci-dessus, |
| $R^9$ | représente un groupe alkyle et |
| E | représente un groupe |

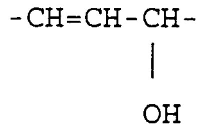

en cas de préparation de lactones, on cyclise les acides carboxyliques,

en cas de préparation des composés éthylène ($A = -CH_2-CH_2$), on soumet à une hydrogénation les composés d'éthène ($A = -CH = CH-$) conformément à des procédés habituels,

et éventuellement on sépare les isomères.